(19) Europäisches Patentamt
European Patent Office
Office européen des brevets

(11) Veröffentlichungsnummer: **0 327 874 B1**

(12) **EUROPÄISCHE PATENTSCHRIFT**

(45) Veröffentlichungstag der Patentschrift: **14.04.93**

(51) Int. Cl.[5]: **C07C 209/22**, C07C 211/48

(21) Anmeldenummer: **89101169.4**

(22) Anmeldetag: **24.01.89**

# (54) Verfahren zur Herstellung von N-Alkylanilinen.

(30) Priorität: **06.02.88 DE 3803662**

(43) Veröffentlichungstag der Anmeldung:
**16.08.89 Patentblatt 89/33**

(45) Bekanntmachung des Hinweises auf die Patenterteilung:
**14.04.93 Patentblatt 93/15**

(84) Benannte Vertragsstaaten:
**BE DE FR GB IT**

(56) Entgegenhaltungen:
**FR-A- 1 070 320**

**CHEMICAL ABSTRACTS, Band 103, 1985, Seite 595, Zusammenfassung Nr. 104685p, Columbus, Ohio, US; & JP-A-60 45 552**

**CHEMICAL ABSTRACTS, Band 54, 1960, Spalte 7599, Zusammenfassung Nr. f, Columbus, Ohio, US; G. ZOLLNER et al.: "Some aspects of ethylation of aniline in the vapor phase"**

(73) Patentinhaber: **BAYER AG**

**W-5090 Leverkusen 1 Bayerwerk(DE)**

(72) Erfinder: **Klug, Günther, Dr.**
**Wiener-Neustädter-Strasse 140**
**W-4019 Monheim 2(DE)**
Erfinder: **Buysch, Hans-Josef, Dr.**
**Brandenburger Strasse 28**
**W-4150 Krefeld(DE)**
Erfinder: **Puppe, Lothar, Dr.**
**Am Weiher 10A**
**W-5093 Burscheid(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 327 874 B1

## Beschreibung

Die vorliegende Erfindung hat ein Verfahren zur Herstellung von N-Alkylanilinen zum Gegenstand, worin man am N-Atom nichtsubstituierte Aniline mit N,N-Dialkylanilinen in Gegenwart von Zeolithen bei erhöhter Temperatur miteinander umsetzt.

N-monoalkylierte Aniline sind wichtige industrielle Zwischenprodukte zur Herstellung von Farbstoffen, Stabilisatoren, Urethanen oder Harnstoffen. Sie werden hergestellt durch Alkylierung von Anilinen mit Alkoholen oder den entsprechenden Dialkylethern in Gegenwart saurer Katalysatoren, beispielsweise in Gegenwart von Phosphoroxychlorid, unter Druck. Dabei entstehen jedoch keine reinen monoalkylierten Aniline, sondern immer auch in beträchtlichem Umfang N,N-Dialkylaniline (Ullmanns Encyclopädie der technischen Chemie, 4. Auflage, Bd. 7 (1974), S. 572 ff.).

Das Verhältnis der monoalkylierten zu den dialkylierten Anilinen, wie es bei derartigen Herstellungsverfahren anfällt, entspricht jedoch nicht immer dem jeweiligen Bedarf. Die monoalkylierten Aniline sind im allgemeinen die begehrteren und gleichzeitig die schwerer zugänglichen Produkte. N,N-Dialkyl-aniline sind zudem nach besonderen Verfahren in guter Ausbeute erhältlich. Daher ist es wünschenswert, das Verhältnis von monoalkylierten zu dialkylierten Anilinen flexibler zu gestalten, d.h. über ein wirksames Verfahren zur Umwandlung von N,N-Dialkyl-anilinen in N-Monoalkyl-aniline zu verfügen. Nach Acta Chim. Hung. 20 - (1959), 321 kann eine Transalkylierung zwischen N,N-Diethyl-anilin und Anilin bewirkt werden, wenn man die beiden Reaktionspartner bei erhöhter Temperatur in der Gasphase über $Al_2O_3$ leitet. Diese Transalkylierung verläuft jedoch sehr langsam. Bei einer Katalysatorbelastung von 0,25 ml/ml Katalysator/h bei 250 °C werden nur 0,16 Mol-% umgewandelt. Erhöht man zur Beschleunigung der Reaktion die Temperatur, wird N,N-Diethylanilin verstärkt umgewandelt, wobei jedoch mehr und mehr kernalkylierte Produkte auftreten und Anilin praktisch nicht am N-Atom alkyliert wird (loc. cit., S. 322, Tab. II). Erhöht man bei 280 °C die Katalysatorbelastung nur geringfügig, geht der Umsatz merklich zurück, und dennoch bilden sich nicht vernachlässigbare Mengen an kernalkylierten Produkten (loc. cit., S. 323, Tab. III).

In JP 60-045 552-A wird vorgeschlagen, die Transalkylierung zwischen N,N-Dimethylanilin und Anilin in Gegenwart von beträchtlichen Mengen an konzentrierter Schwefelsäure mit einem großen Überschuß an Anilin bei 180° bis 300 °C im Autoklaven durchzuführen. Diese Reaktion nimmt mehrere Stunden in Anspruch; der $H_2SO_4$-Katalysator muß mit Alkali neutralisiert (entsorgt) werden und geht verloren; schließlich ist der Umsatz trotz hoher Katalysatormenge und Anilinüberschuß noch unvollständig. Darüber hinaus ist eine Druckreaktion grundsätzlich mit besonderem technischen Aufwand und im vorliegenden Fall auch mit Korrosionsgefahren verbunden.

Es wurde nun gefunden, daß man die Transalkylierung deutlich rascher und selektiver in Gegenwart von Alumosilikaten von Zeolithtyp bei Temperaturen zwischen 200 und 350 °C durchführen kann. Bei hohen Umsätzen verläuft die Reaktion glatt und praktisch ohne Kernalkylierung.

Es wurde demnach ein Verfahren zur Herstellung von N-Alkylanilinen der Formel

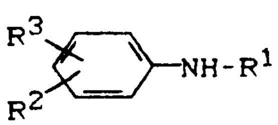 (I)

durch Umsetzung von Anilinen mit N,N-Dialkyl-anilinen der Formeln

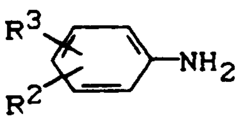 (II) bzw.

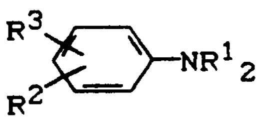 (III),

wobei in den Formeln

$R^1$ $C_1$-$C_4$-Alkyl bedeutet und

$R^2$ und $R^3$ unabhängig voneinander für Wasserstoff, $C_1$-$C_4$-Alkyl, $C_1$-$C_4$-Alkoxy, Fluor, Chlor oder Brom stehen,

in der Gas- oder Sumpfphase bei erhöhten Temperaturen gefunden, das dadurch gekennzeichnet ist, daß man die Umsetzung in Gegenwart von Alumosilikaten vom Zeolithtyp bei Temperaturen von 200 bis 350 °C, bevorzugt 220 bis 330 °C, besonders bevorzugt 230 bis 320 °C, durchführt.

Bevorzugte Zeolithe sind kristalline Alumosilikate mit folgender allgemeiner oxidischer Formel:

$M_{m/z}[mAlO_2 \cdot nSiO_2] \cdot qH_2O$ (IV),

worin

- n/m das Si/Al-Verhältnis darstellt,
- $M_{m/z}$ austauschbare Kationen bedeutet, wobei
- z die Wertigkeit des Kations angibt, und
- q die Menge der sorbierten Wasserphase angibt.

Verschiedene Zeolith-Typen bzw. -Strukturen sind beispielsweise in D.W. Breck, Zeolite Molecular Sieves, John Wiley and Sons Inc., New York 1974 beschrieben. In solchen Zeolithen kann weiterhin das Aluminium teilweise durch andere dreiwertige Ionen wie z.B. Fe(III), B (III) oder Ga (III) ersetzt sein.

Bei synthetischen Zeolith-Strukturtypen, die man durch Verwendung von organischen Zusatzstoffen erhält, ist vor der Herstellung der erfindungsgemäß einsetzbaren ausgetauschten Formen eine Calcinierung zur Entfernung organischer Reste erforderlich. Die H-Form der erfindungsgemäßen Zeolithe kann durch Ammoniumaustausch und anschließende Kalzinierung bzw. durch Eintausch von Wasserstoffionen mit Hilfe von Mineralsäuren hergestellt werden. Andere Kationen in erfindungsgemäß einsetzbaren Zeolithen sind beispielsweise: $Na^{\oplus}$, $K^{\oplus}$, $Li^{\oplus}$, $Ca^{2+}$, $Mg^{2+}$, $Ba^{2+}$, $Zn^{2+}$, $Cu^{2+}$, $Ag^{+}$, $Ga^{3+}$, $La^{3+}$, $Ce^{3+}$, $Fe^{3+}$, $Ni^{2+}$, $Sn^{2+}$, $Ti^{4+}$, $Zr^{4+}$, $Mn^{2+}$, $Cr^{3+}$, $Co^{2+}$, bevorzugt $Na^{+}$, $K^{+}$, $Li^{+}$, $Ca^{2+}$, $Mg^{2+}$, $Zn^{2+}$, $Cu^{2+}$, $La^{3+}$, $Ce^{3+}$, $Fe^{3+}$, $Ni^{2+}$, $Sn^{2+}$.

Erfindungsgemäß werden insbesondere Zeolithe mit einem Si/Al-Verhältnis n/m $\geq 1$ eingesetzt. Weiterhin werden erfindungsgemäß Zeolithe mit einem Porendurchmesser $\geq 5$Å eingesetzt.

Unter die Formel (IV) fallen beispielsweise Zeolithe vom Strukturtyp des Zeolith L, Faujasit, Mazzit, Mordenit, Offretit, Cancrinit, Gmelinit, ZSM 12, ZSM 25, Ferrierit, Zeolith $\beta$, Zeolith Y, ZSM 5, ZSM 11, ZSM 22, ZSM 23, ZSM 48, ZSM 43, PSH 3 und Heulandit. Bevorzugt unter diesen Strukturtypen sind Faujasit, Zeolith L, ZSM 12 und Mordenit. Besonders bevorzugt sind Zeolithe vom Strukturtyp Faujasit (wie z.B. Zeolith Y) und Zeolith L.

Da die $H^{+}$-Formen solcher Zeolithe saure Eigenschaften aufweisen, sind sie katalytisch besonders wirksam. Erfindungsgemäß bevorzugt sind daher weiterhin Zeolithe der obengenannten Strukturtypen, bei denen ein Teil der austauschbaren Kationen Wasserstoffionen bedeuten, wobei bevorzugt über 50 Äquivalent-%, besonders bevorzugt mindestens 80 Äquivalent-%, der austauschbaren Kationen Wasserstoffionen sind. Dier Herstellung solcher teilweise sauren Formen von Zeolithen mit Hilfe von starken Säuren ist Stand der Technik und braucht daher nicht weiter dargelegt zu werden. Beschrieben werden derartige Verfahren beispielsweise in J.A. Rabo, Zeolite Chemistry and Catalysis, ACS Monograph 171, Washington D.C. 1976.

Die erfindungsgemäße Transalkylierung kann so durchgeführt werden, daß man die Reaktionspartner Anilin und N,N-Dialkyl-anilin bzw. ihre kernsubstituierten Derivate bei Temperaturen ab 200 °C gasförmig, gegebenenfalls unter Verwendung eines inerten Trägergases, wie $N_2$, über einen der genannten Zeolithkontakte leitet. Die Katalysatorbelastung kann 0,1 bis 8, bevorzugt 0,2 bis 5, besonders bevorzugt 0,3 bis 4, Liter umzusetzendes Gemisch pro Liter Zeolith pro Stunde eingestellt werden. Die Reaktion kann jedoch ebenfalls in der Sumpfphase bei Temperaturen ab 200 °C unter dem sich einstellenden Eigendruck durchgeführt werden. Hierbei beträgt die Menge des Zeolithkontaktes 2 - 50 Gew.-%, bevorzugt 5 - 40 Gew.-%, besonders bevorzugt 7 - 30 Gew.-%, bezogen auf das Gesamtgewicht des Ansatzes. Die Variante des erfindungsgemäßen Verfahrens mit Umsetzung in der Gasphase ist besonders elegant, weil sie im allgemeinen drucklos durchgeführt werden kann, und ist daher bevorzugt.

Für den Betrieb in Gasphasenreaktoren werden die erfindungsgemäß einzusetzenden Zeolithe in stückige Form gebracht. Hierzu werden sie mit Bindern verpreßt und granuliert. Als Bindermaterialien werden vorzugsweise $\gamma$-$Al_2O_3$ und $SiO_2$, beispielsweise in Mengen von 15 bis 50 Gew.-%, bezogen auf den fertigen Zeolith-Katalysator, verwendet.

Das Molverhältnis der Reaktionspartner ist nicht kritisch und kann 0,3 bis 5, bevorzugt 0,5 bis 3, besonders bevorzugt 0,8 bis 1,5, Mol Anilin zu Mol N,N-Dialkylanilin betragen.

Es ist weiterhin möglich, inerte Lösungsmittel mit zu verwenden, beispielsweise Kohlenwasserstoffe wie Benzol, Toluol, Xylol, Benzin, Cyclohexan, Decalin und/oder Isooctan.

Die umzusetzenden Ausgangsmaterialien für das erfindungsgemäße Verfahren sind solche der Formeln (II) und (III). Bevorzugte Ausgangsstoffe sind solche, in denen $R^{11}$ mit der Bedeutung $C_1$-$C_2$-Alkyl anstelle von $R^1$ tritt. Weitere bevorzugte Ausgangsstoffe sind solche, in denen $R^{12}$ und $R^{13}$ mit der voneinander unabhängigen Bedeutung Wasserstoff, $C_1$-$C_2$-Alkyl, $C_1$-$C_2$-Alkoxy, Fluor oder Chlor oder besonders bevorzugt $R^{22}$ und $R^{23}$ mit der voneinander unabhängigen Bedeutung Wasserstoff oder $C_1$-$C_2$-Alkyl an die Stelle von $R^2$ und $R^3$ treten. Weitere bevorzugte Ausgangsstoffe sind solche, in denen $R^3$ Wasserstoff ist.

Beispiel 1

Je 0,5 mol Anilin und N,N-Dimethylanilin wurden in 200 ml Benzol gelöst und mit 20 g eines der genannten Zeolithen 3 Stunden bei 300 °C im Autoklaven erhitzt. Die Bildung von N-Methylanilin gibt die folgende Tabelle I wieder:

Tabelle I

| Nr. | Zeolith | N-Methylanilin (Gew.-%) |
|---|---|---|
| 1.1 | H-ZSM 5 | 21 |
| 1.2 | H-ZSM 11 | 27 |
| 1.3 | Sn-ZSM 5 | 14 |
| 1.4 | H-Mordenit | 32 |
| 1.5 | H-Y | 49 |

Die Tabelle I gibt die Ergebnisse der Transalkylierung wieder, die bei absatzweiser Durchführung erhalten werden und zeigt auch die herausragende Katalytische Wirkung der Zeolithe besonders von H-ZSM 11, H-Mordenit und besonders H-Y.

Beispiel 2

In ein Reaktionsrohr von ca. 20 mm Durchmesser wurden 20 g eines Zeolithgranulats mit je ca. 30 Gew.-% Binder und einem mittleren Korndurchmesser von 1 bis 2 mm eingefüllt. Ein Gemisch von Anilin und N,N-Dimethylanilin wurde verdampft und das Dampfgemisch bei unterschiedlichen Temperaturen über die Katalysatorschüttung geleitet. Die folgende Tabelle II gibt die Umwandlung in N-Methylanilin wieder. Die Zusammensetzung der Reaktionsgemische wurde gaschromatographisch ermittelt.

**Tabelle II**

| Nr. | Katalysator | Molverhältnis A/DMA 2) | °C | h | Belastung ml/ml/h | Gew.-% 2) A | NMA | DMA | kernalkyl. |
|---|---|---|---|---|---|---|---|---|---|
| 2.1 | H-ZSM 11/γ-$Al_2O_3$ | 3:1 | 300 | 4 | 1,0 | 64 | 13 | 23 | 0 |
| 2.2 | H-Y/γ-$Al_2O_3$ | 1:1 | 250 | 4 | 1,0 | 28 | 31 | 41 | <0,1 |
| 2.3 | H-L/γ-$Al_2O_3$ | 1:1 | 250 | 4 | 1,0 | 29 | 29 | 42 | <0,3 |
| 2.4 | H-Y/$SiO_2$ | 1:1 | 250 | 4 | 1,0 | 30 | 27 | ~43 | ~0,5 |
| 2.5 | γ-$Al_2O_3$ 1) | 1:1 | 300 | 8 | 1,0 | 42 | 1 | 57 | 0 |
| 2.6 | $SiO_2$ 1) | | 300 | | keine Umwandlung | | | | |

1) Diese Materialien wurden als Binder für obige Zeolithe verwendet.

2) A = Anilin; NMA = N-Methylanilin; DMA = N,N-Dimethylanilin.

Nach Tabelle II wurden also bei weit höherer Katalysatorbelastung als nach dem Stand der Technik hohe Transalkylierungsraten erzielt. Schon bei 250 °C wurde auch ohne Anilinüberschuß ein großer Teil, nämlich bis 40 Mol-% des Dimethylanilins umgesetzt. Dabei entstanden praktisch keine kernalkylierten Produkte. Mit den Materialien ($SiO_2$, γ-$Al_2O_3$), die als Binder für die Zeolithe verwendet wurden, konnte unter den Bedingungen der Erfindung keine ($SiO_2$) oder nur eine sehr geringe (γ-$Al_2O_3$) Transalkylierung

bewirkt werden. Die Versuche 2.5 und 2.6 sind daher Vergleichsversuche.

Beispiel 3

N,N-Diethylanilin und Anilin wurden im Molverhältnis 1:1 wie in Beispiel 2 über einen Zeolith-Kontakt geleitet. Die folgende Tabelle III gibt das Resultat wieder:

Tabelle III

| Nr. | Katalysator | Temperatur °C | Belastung ml/ml/h | Produktverteilung Gew.-% 1) A | NEA | DEA | kernalkyl. |
|---|---|---|---|---|---|---|---|
| 3.1 | H-Y/$SiO_2$ | 250 | 1,0 | 26 | 22 | 51 | <1 % |
| 3.2 | H-Y/γ-$Al_2O_3$ | 250 | 1,0 | 25 | 26 | 48 | <1 % |
| 3.3 | H-Y/$SiO_2$ | 300 | 1,0 | 19 | ~59 | 19 | 3,1 nach 1 h |
| 3.4 | H-Y/$SiO_2$ | 300 | 1,0 | 19 | ~59 | 21 | 1,5 nach 5 h |

1) A = Anilin; NEA = N-Ethylanilin; DEA = N,N-Diethylanilin.

Man erkennt auch hier eine hohe Selektivität der Y-Zeolithe bezüglich der Bildung von N-Ethylanilin. Der Anteil an kernalkylierten Verbindungen, der in einigen Fällen zu Beginn der Reaktion verhältnismäßig

hoch ist, geht innerhalb kurzer Zeit auf unbedeutende Werte zurück, ohne daß der Umsatz ansonsten beeinträchtigt wird. Dies verdeutlichen die Angaben unter 3.3 und 3.4, wo während der 5-stündigen Laufzeit der Kernalkylanteil von 3,1 auf 1,5 Gew.-% zurückging.

## Patentansprüche

1. Verfahren zur Herstellung von N-Alkylanilinen der Formel

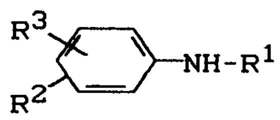

durch Umsetzung von Anilinen mit N,N-Dialkylanilinen der Formeln

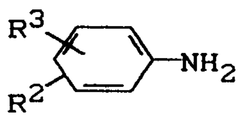

bzw.

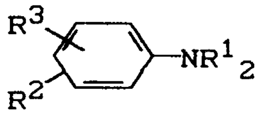

,

wobei in den Formeln

| | |
|---|---|
| $R^1$ | $C_1$-$C_4$-Alkyl bedeutet und |
| $R^2$ und $R^3$ | unabhängig voneinander für Wasserstoff, $C_1$-$C_4$-Alkyl, $C_1$-$C_4$-Alkoxy, Fluor, Chlor oder Brom stehen, |

in der Gas- oder Sumpfphase bei erhöhten Temperaturen, dadurch gekennzeichnet, daß man die Umsetzung in Gegenwart von Alumosilikaten vom Zeolithtyp bei Temperaturen von 200 bis 350 °C, bevorzugt 220 bis 330 °C, besonders bevorzugt 230 bis 320 °C, durchführt.

2. Verfahren nach Anspruch 1, dadurch gekennzeichnet, daß als Zeolithe solche der Formel:

$$M_{m/z}[mAlO_2 \cdot nSiO_2] \cdot qH_2O$$

eingesetzt werden, worin

| | |
|---|---|
| n/m | das Si/Al-Verhältnis darstellt, |
| $M_{m/z}$ | austauschbare Kationen bedeutet, wobei |
| z | die Wertigkeit des Kations angibt, und |
| q | die Menge der sorbierten Wasserphase angibt. |

3. Verfahren nach Anspruch 2, dadurch gekennzeichnet, daß Zeolithe mit einem Si/Al-Verhältnis n/m ≧1 eingesetzt werden.

4. Verfahren nach Anspruch 1, dadurch gekennzeichnet, daß Zeolithe mit einem Porendurchmesser ≧5Å eingesetzt werden.

5. Verfahren nach Anspruch 2, dadurch gekennzeichnet, daß als Zeolithe solche vom Strukturtyp Zeolith L, Faujasit, Mazzit, Mordenit, Offretit, Cancrinit, Gmelinit, ZSM 12, ZSM 25, Ferrierit, Zeolith $\beta$, Zeolith Y, ZSM 5, ZSM 11, ZSM 22, ZSM 23, ZSM 48, ZSM 43, PSH 3 oder Heulandit eingesetzt werden.

6. Verfahren nach Anspruch 5, dadurch gekennzeichnet, daß als Zeolithe solche vom Strukturtyp Faujasit, Zeolith $\beta$, Zeolith L, ZSM 12 oder Mordenit eingesetzt werden.

7. Verfahren nach Anspruch 6, dadurch gekennzeichnet, daß als Zeolithe solche vom Strukturtyp Faujasit und Zeolith L eingesetzt werden.

8. Verfahren nach Anspruch 2, dadurch gekennzeichnet, daß ein Teil der austauschbaren Kationen Wasserstoffionen bedeuten, bevorzugt >50 Äquivalent-%, besonders bevorzugt ≧80 Åquivalent-% aller austauschbaren Kationen.

9. Verfahren nach Anspruch 1, dadurch gekennzeichnet, daß bei einer Katalysatorbelastung von 0,1 bis 8, bevorzugt 0,2 bis 5, besonders bevorzugt 0,3 bis 4, Liter umzusetzendes Gemisch/l Zeolith/h gearbeitet wird.

10. Verfahren nach Anspruch 1, dadurch gekennzeichnet, daß ein Molverhältnis von 0,3 bis 5, bevorzugt 0,5 bis 3, besonders bevorzugt 0,8 bis 1,5, Mol Anilin/Mol N,N-Dialkyl-anilin im umzusetzenden Gemisch eingestellt wird.

**Claims**

1. Process for the preparation of N-alkylanilines of the formula

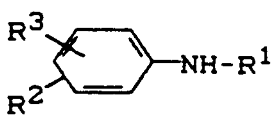

by reaction of anilines with N,N-dialkylanilines of the formulae

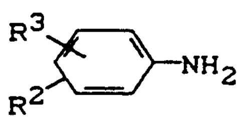

or

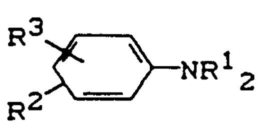

in which formulae

$R^1$ denotes $C_1$-$C_4$-alkyl and

$R^2$ and $R^3$, independently of one another, stand for hydrogen, $C_1$-$C_4$-alkyl, $C_1$-$C_4$-alkoxy, fluorine, chlorine or bromine,

in the gas or liquid phase at elevated temperatures, characterised in that the reaction is carried out in the presence of aluminosilicates of the zeolite type at temperatures of 200 to 350 ° C, preferably 220 to 330 ° C, particularly preferably 230 to 320 ° C.

2. Process according to Claim 1, characterised in that the zeolites used are those of the formula

$M_{m/z}[mAlO_2 \cdot nSiO_2] \cdot qH_2O$

in which

n/m represents the Si/Al ratio,

$M_{m/z}$ denotes exchangeable cations, in which
z indicates the valence of the cation and
q indicates the amount of the adsorbed water phase.

**3.** Process according to Claim 2, characterised in that the zeolites having an Si/Al ratio n/m ≧1 are used.

**4.** Process according to Claim 1, characterised in that zeolites having a pore diameter ≧5Å are used.

**5.** Process according to Claim 2, characterised in that the zeolites used are those of the structure type zeolite L, faujasite, mazzite, mordenite, offretite, cancrinite, gmelinite, ZSM 12, ZSM 25, ferrierite, zeolite $\beta$, zeolite Y, ZSM 5, ZSM 11, ZSM 22, ZSM 23, ZSM 48, ZSM 43, PSH 3 or heulandite.

**6.** Process according to Claim 5, characterised in that the zeolites used are those of the structure type faujasite, zeolite $\beta$, zeolite L, ZSM 12 or mordenite.

**7.** Process according to Claim 6, characterised in that the zeolites used are those of the structure type faujasite or zeolite L.

**8.** Process according to Claim 2, characterised in that a portion of the exchangeable cations denote hydrogen ions, preferably >50 equivalent %, particularly preferably ≧80 equivalent %, of all exchangeable cations.

**9.** Process according to Claim 1, characterised in that the reaction is carried out at a space velocity of the catalyst of 0.1 to 8, preferably 0.2 to 5, particularly preferably 0.3 to 4, litres of mixture to be reacted/l of zeolite/h.

**10.** Process according to Claim 1, characterised in that the molar ratio is set to 0.3 to 5, preferably 0.5 to 3, particularly preferably 0.8 to 1.5, mol of aniline/mol of N,N-dialkylaniline in the mixture to be reacted.

**Revendications**

**1.** Procédé de production de N-alkylanilines de formule

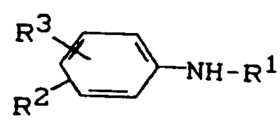

par réaction d'anilines avec des N,N-dialkylanilines de formules

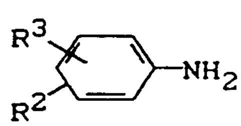

ou

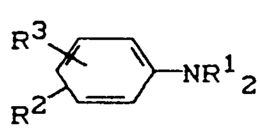

,

formules dans lesquelles
$R^1$ désigne un groupe alkyle en $C_1$ à $C_4$, et
$R^2$ et $R^3$ représentent indépendamment l'un de l'autre l'hydrogène, un groupe alkyle en $C_1$ à $C_4$,

alkoxy en $C_1$ à $C_4$, le fluor, le chlore ou le brome,
en phase gazeuse ou en phase pâteuse à des températures élevées, caractérisé en ce qu'on conduit la réaction en présence d'aluminosilicates de type zéolitique à des températures de 200 à 350° C, de préférence de 220 à 330° C, notamment de 230 à 320° C.

**2.** Procédé suivant la revendication 1, caractérisé en ce qu'on utilise comme zéolites des zéolites de formule

$$M_{m/z}[mAlO_2 \cdot nSiO_2] \cdot qH_2O$$

dans laquelle
le rapport n/m représente le rapport si/Al,

| | |
|---|---|
| $M_{m/z}$ | désigne des cations échangeables, |
| z | indiquant la valence du cation et |
| q | indiquant la quantité de phase aqueuse fixée par sorption. |

**3.** Procédé suivant la revendication 2, caractérisé en ce qu'on utilise des zéolites avec un rapport n/m du silicium à l'aluminium supérieur ou égal à 1.

**4.** Procédé suivant la revendication 1, caractérisé en ce qu'on utilise de la zéolite ayant un diamètre des pores supérieur ou égal à 5 Å.

**5.** Procédé suivant la revendication 2, caractérisé en ce qu'on utilise comme zéolites des zéolites du type structural de la zéolite L, de la faujasite, de la mazzite, de la mordénite, de l'offretite, de la cancrinite, de la gmélinite, de ZSM 12, de ZSM 25, de la ferriérite, de la zéolite $\beta$, de la zéolite Y, de ZSM 5, de ZSM 11, de ZSM 22, de ZSM 23, de ZSM 48, de ZSM 43, de PSH 3 ou d'heulandite.

**6.** Procédé suivant la revendication 5, caractérisé en ce qu'on utilise comme zéolites des zéolites du type structural de la faujasite, de la zéolite $\beta$, de la zéolite L, de ZSM 12 ou de la mordénite.

**7.** Procédé suivant la revendication 6, caractérisé en ce qu'on utilise comme zéolites des zéolites du type structural de la faujasite ou de la zéolite L.

**8.** Procédé suivant la revendication 2, caractérisé en ce qu'une partie des cations échangeables représente des ions hydrogène, de préférence plus de 50 équivalents %, notamment une proportion égale ou supérieure à 80 équivalents %, de tous les cations échangeables.

**9.** Procédé suivant la revendication 1, caractérisé en ce qu'on opère avec une charge de catalyseur de 0,1 à 8, de préférence de 0,2 à 5, notamment 0,3 à 4 litres de mélange en réaction par litre de zéolite par heure.

**10.** Procédé suivant la revendication 1, caractérisé en ce qu'on établit un rapport molaire de 0,3 à 5, de préférence de 0,5 à 3, notamment de 0,8 à 1,5 mole d'aniline par mole de N,N-dialkyl-aniline dans le mélange en réaction.